# EUROPEAN PATENT SPECIFICATION

(11) **EP 2 489 316 B1**
(45) Date of publication and mention of the grant of the patent: **01.10.2014**
(21) Application number: 12155276.4
(22) Date of filing: 14.02.2012
(51) Int. Cl.: A61B 17/34, A61B 17/00

(54) **Compliant access device including proximal adhesive patch**
Konforme Zugangsvorrichtung mit proximalem Haftpflaster
Dispositif d'accès souple incluant un timbre adhésif proximal

(30) Priority: 15.02.2011 US 201161442859 P; 31.01.2012 US 201213362021
(43) Date of publication of application: 22.08.2012
(73) Proprietor: Covidien LP, Mansfield, MA 02048 (US)
(72) Inventor: Pribanic, Russell, Roxbury, CT 06783 (US); Kasvikis, Dino, Mansfield, MA 02048 (US); Fischvogt, Gregory, Hamden, CT 06514 (US)
(74) Representative: Soames, Candida Jane

(56) References cited:
- EP-A1- 0 577 400
- EP-A1- 2 044 889
- EP-A1- 2 138 117
- EP-A2- 2 138 118
- WO-A2-2009/047707
- US-A- 5 683 378

## Description

### 1. Technical Field

The present disclosure relates to an access device for use in a surgical procedure. More particularly, the present disclosure relates to an access device adapted for sealed and stable insertion into a tissue tract.

### 2. Background

In an effort to reduce trauma and recovery time, many surgical procedures are performed through small openings in the skin, such as an incision or a natural body orifice, as compared to the larger incisions typically required in traditional procedures. Generally, such procedures are referred to as "endoscopic", unless performed on the patient's abdomen, in which case the procedure is referred to as "laparoscopic". Throughout the present disclosure, the term "minimally invasive" should be understood to encompass any and all such procedures.

During a typical minimally invasive procedure, surgical objects, such as surgical access devices, e.g., trocar and cannula assemblies, or endoscopes, are inserted into the patient's body through an incision in tissue. Prior to the introduction of the surgical object into the patient's body, insufflation gases may be used to enlarge the area surrounding the target surgical site to create a larger, more accessible work area. Accordingly, the maintenance of a substantially fluid-tight seal is desirable so as to prevent the escape of the insufflation gases and the deflation or collapse of the enlarged surgical site.

To this end, access devices are configured in a variety of ways to secure and seal the same within tissue. For example, the access device may be shaped, e.g., in an hour glass shape, or the end of the device which projects into tissue may be expandable or insufflatable thereby providing a fixation force inside the tissue for securing and sealing the access device thereto.
US 5683378 discloses an access device according to the pre-amble of Claim 1. However, a continuing need exists for an access device that can be customized to accommodate varying tissue wall thicknesses and be inserted directly therein, and that can accommodate a variety of surgical objects while maintaining the integrity of an insufflated workspace.

### SUMMARY

As provided by Claim 1 there is disclosed a compliant access device for positioning within a tissue tract for accessing an underlying body cavity having a proximal portion, a distal portion, a body portion interconnecting the proximal and distal portions, and at least one port extending therethrough. The proximal portion includes a tissue facing surface and defines a first radial dimension. The distal portion extends longitudinally along an axial length and defines a second radial dimension that is substantially uniform along the axial length and smaller than the first radial dimension of the proximal portion. An adhesive is disposed on the tissue facing surface of the proximal portion for releasably securing and sealing the proximal portion to a tissue surface.

The proximal portion exhibits an arcuate configuration.

The adhesive may be selected from acrylics, silicones, urethanes, and hydrogels. In embodiments, the adhesive is activated or de-activated by an external stimulus. The external stimulus may be selected from heat, light, and fluid. In embodiments, the adhesive may be pressure sensitive.

The adhesive may be coated on the tissue facing surface of the proximal portion or may be a layer that is affixed to the tissue facing surface of the proximal portion. In embodiments, the layer may further include concave surfaces or gecko feet for mechanically securing the tissue facing surface of the proximal portion of the tissue surface.

### BRIEF DESCRIPTION OF THE DRAWINGS

Various embodiments of the present disclosure are described hereinbelow with references to the drawings, wherein:
FIG. 1 is a front perspective view of an access device in accordance with an embodiment of the present disclosure positioned relative to the tissue;
FIG. 2 is a cross-sectional view of the access device of FIG. 1 positioned within the tissue;
FIG. 3 is a front perspective view of an access device including a textured adhesive layer in accordance with the present disclosure;
FIG. 4 is a front perspective view of an access device including a texture adhesive layer in accordance with the present disclosure;
FIG. 5 is a front perspective view of an access device in accordance with the present disclosure;
FIG. 6 is a top view of the access device of FIG. 5; and
FIG. 7 is a cross-sectional view of the access device of FIG. 5 taken along line 7-7 of FIG. 5 illustrating a port that extends longitudinally therethrough.

### DETAILED DESCRIPTION OF THE EMBODIMENTS

Various embodiments of the presently disclosed access device, and methods of using the same, will now be described in detail with reference to the drawings wherein like reference numerals identify similar or identical elements. In the drawings, and in the following description, the term "proximal" should be understood as referring to the end of the access device, or component thereof, that is closer to the clinician during proper use, while the term "distal" should be understood as referring to the end that is farther from the clinician, as is traditional and conventional in the art.

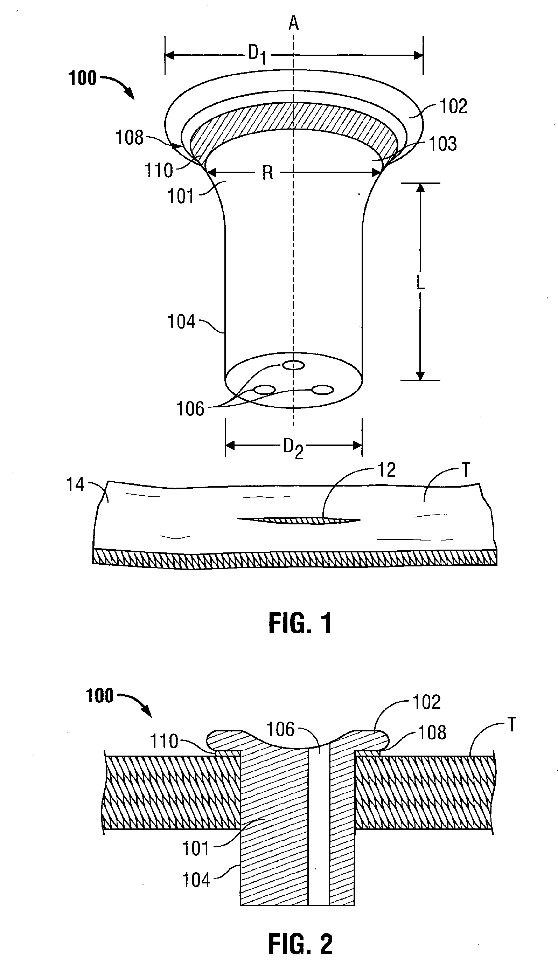

With reference to FIG. 1, an access device 100 for use in a surgical procedure, e.g., a minimally invasive procedure, is illustrated. Access device 100 is configured and adapted to be inserted within a tissue tract 12 defined by tissue surface 14 formed in tissue "T", e.g., an incision. Although the presently described access device 100 is discussed in connection with minimally invasive procedures, it is within the scope of the present disclosure that the access device 100 may be used through a naturally occurring opening or any incision in a patient's skin.

Access device 100 includes a body portion 101 extending between a proximal portion 102 and a distal portion 104. Proximal and distal portions 102, 104 may be monolithically formed with body portion 101, such as by molding, or may be secured to body portion 101 by conventions means, such as for example, ultrasonic welding or via the use of adhesives. Access device 100 includes one or more ports or lumens 106 that extend longitudinally along the length of the access device 100 through proximal and distal portions 102, 104. Ports 106 are adapted to receive a surgical object, such as a surgical instrument, in a substantially sealed relation. Examples of surgical instrumentation which may be introduced through ports 106 of the access device 100 include clip appliers, graspers, dissectors, retractors, staplers, laser probes, photographic devices, endoscopes, laparoscopes, arthroscopes, tubes, electrosurgical cutting, coagulating, and ablation devices, and other tools within the purview of those skilled in the art.

Access device 100 is constructed from a non-degradable, medical-grade material, such as plastic and/or elastomeric materials. In embodiments, the access device 100 may be fabricated from a soft synthetic resin, such as polyurethane or silicone. In other embodiments, access device 100 may be formed from a foam material having sufficient compliance to form a seal about one or more surgical objects and also establish a sealing relation with the tissue. The foam may be sufficiently compliant to accommodate off axis motion of a surgical object (not shown) inserted through port 106. In yet other examples, access device 100 may be formed of a rigid material such as polymeric materials like acrylonitrile-butadiene-styrene, polycarbonate, and polystyrene.

Proximal portion 102 of access device 100 defines a first diameter D₁ and distal portion 104 defines a second diameter D₂. As illustrated in the current embodiment, the first diameter D₁ of proximal portion 102 is larger than the second diameter D₂ of distal portion 104 thereby defining a goose-neck or tapering portion 103 within body portion 101 for interconnecting the proximal and distal portions 102, 104. Thus, the body portion 101 defines a radial dimension "R" that varies along the length thereof. The goose-neck or tapering portion 103 adjacent the proximal portion 102 facilitates the anchoring of access device 100 within tissue "T" as the radial dimension "R" is appreciably less than the diameter D₁ of proximal portion 102 such that access device 100 defines an arcuate shape or configuration.

The second diameter D₂ of distal portion 104 is substantially uniform along a length "L" such that the access device 100 may be easily placed within tissue. The substantially uniform length assists in the insertion of access device 100 within tissue tract 12 defined by tissue surface 14 and formed in tissue "T". The substantially uniform second diameter "D₂" allows for the length "L" of distal portion 104 to be trimmed to a desired length depending upon the thickness of the tissue "T" in which the access device 100 is to be placed without affecting the integrity or function of the access device 100. Alternatively, the diameter "D₂" of distal portion 104 may vary along the axial dimension to facilitate the anchoring of the access device 100 within tissue "T". In cross section, distal portion 102 may exhibit any suitable configuration, e.g., substantially circular, oval or oblong.

Each port 106 is configured to removably receive a surgical object (not shown). Port 106 may be an open channel extending along the length of the access device 100 as illustrated in FIG. 2. The diameter of port 106 may be about 5 mm to about 15 mm, as these dimensions are typical of the surgical objects used during the course of minimally invasive procedures. However, access device 100 including ports 106 of substantially larger, or smaller, diameters is not beyond the scope of the present disclosure. In embodiments, such as those utilizing a soft or flexible material, each port 106 may be provided in a first state which is closed or of a sufficiently small dimension such that the escape of insufflation gas through the port 106 in the absence of a surgical objection is substantially prevented. Upon the introduction of a surgical object into port 106, the port 106 transitions or is stretched to a second state which defines a second, larger dimension that substantially approximates or conforms to the diameter of the surgical object such that a substantially fluid-tight seal is formed therewith, thereby substantially preventing the escape of insufflation gas through port 106 of access device 100 in the presence of the surgical object. Alternatively, access device 100 may be devoid of ports 106. With this arrangement, ports 106 are created within access device 100 during the insertion of a surgical object. In accordance with this embodiment, access device 100 is formed of a flowable or sufficiently compliable material such as a foam material, e.g., an open-cell polyurethane foam or a gel.

As depicted in FIGS. 1 and 2, proximal portion 102 defines a substantially arcuate shape that is configured to be larger than the distal portion 104 in order to engage tissue "T" and prevent the access device 100 from going through tissue "T". Proximal portion 102 includes a tissue facing surface 108 including an adhesive 110 to facilitate the securement of the access device 100 to the tissue surface 14 and thus, within the tissue tract 12 in tissue "T". Adhesive 110 must firmly, yet temporary, adhere and seal the access device 100 to the tissue "T" surrounding the tissue tract 12. The adhesive 110 should also be acceptable for use on skin without contact deterioration (for example, the adhesive should preferably be non-irritating and non-sensitizing). Typical adhesives can include acrylics, silicone, urethanes, hydrogels, and the like. Additionally, the adhesive could be activated or de-activated by an external stimulus such as heat, light, or a given fluid solution or chemical reaction such that the access device 100 may be sealingly bonded to tissue "T" yet be detachable so that the access device 100 may be removed upon completion of use. Adhesive 110 may be disposed on the entire tissue facing surface 108 in an annular or "donut" shape, or may be applied on an outer peripheral region of the tissue facing surface 108. It is envisioned that a variety of patterns may be utilized so long as the adhesive is disposed continuously around the tissue facing surface 108 of proximal portion 102 thereby effecting a seal about tissue "T".

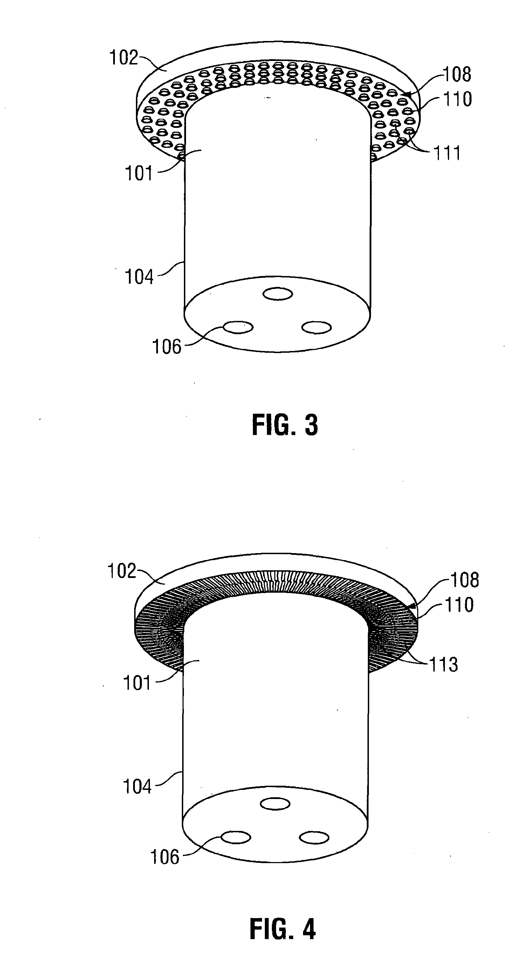

In an embodiment, adhesive 110 may be coated on the tissue facing surface 108 of the proximal portion 102 of the access device 100. In other embodiments, the adhesive 110 is a layer of adhesive material which is attached to tissue facing surface 108 or provided as a separate piece that is configured for positioning between the tissue facing surface 108 and tissue "T", e.g., a double sided tape. Releasable contact liners (not shown) may be utilized to protect the adhesive 110 prior to use. In embodiments, the adhesive 110 may be pressure-sensitive so that it forms immediate attachments on contact with tissue "T". In some embodiments, the adhesive 110 may be a textured layer including concave surfaces 111 (FIG. 3) or gecko feet 113 (FIG. 4) which act as suction pads to mechanically aid in releasably securing the access device 100 to tissue "T". Alternatively, adhesive 110 may be a liquid substance applied to the tissue facing surface 110 or to tissue "T" prior to inserting the access device 100 within tissue tract 12.

Referring again to FIGS. 1 and 2, the use of access device 100 will be discussed during the course of a typical minimally invasive procedure. Initially, the peritoneal cavity (not shown) is insufflated with a suitable biocompatible gas such as, e.g., CO₂ gas, such that the cavity wall is raised and lifted away from the internal organs and tissue housed therein, providing greater access thereto. The insufflation may be performed with an insufflation needle or similar device, as is conventional in the art. Either prior or subsequent to insufflation, a tissue tract 12 is created in tissue "T", the dimensions of which may be varied dependent upon the nature of the procedure.

Prior to the insertion of access device 100 within tissue tract 12, the distal portion 104 may be trimmed to the desired length. The distal portion 104 is then inserted into tissue tract 12 until tissue facing surface 108 of proximal portion 102 abuts tissue surface 14. Adhesive 110 contacts tissue "T" thereby creating a substantially fluid-tight seal between the access device 100 and tissue surface 14 and substantially preventing the escape of insufflation gas around access device 100 and through tissue tract 12. This configuration obviates the need for the hour glass configuration or enlarged distal end typically required to generally anchor and seal the access device 100 within tissue "T".

After successfully anchoring access device 100 within the patient's tissue "T", one or more surgical objects may be inserted through one or more ports 106. After use of the access device 100, the access port 100 may be removed from the tissue tract 12 by detaching the adhesive 110 from tissue surface 14 via application of an external force or stimuli thereby allowing for the extraction of the distal portion 104 from tissue "T".

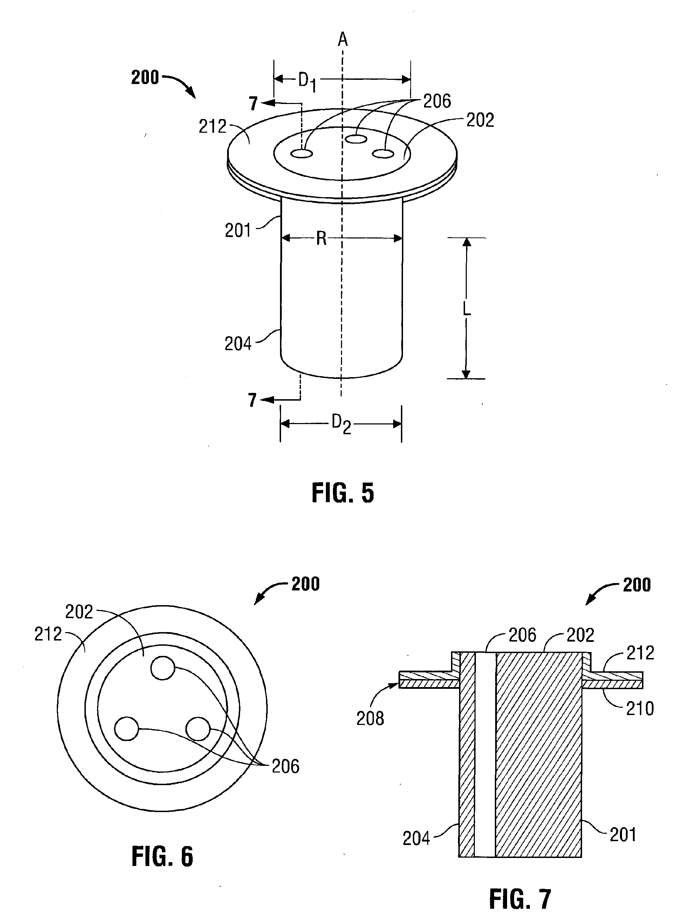

With reference now to FIGS. 5-7, another example of access device 200 is disclosed. Access device 200 includes a body portion 201 that extends along a longitudinal axis "A". The body portion 201 of access device 200 defines a radial dimension "R" that is substantially uniform with the diameters D1, D2 of proximal and distal portions 202, 204, respectively, such that the access device 200 has substantially uniform dimensions along the length thereof. As described above, distal portion 204 is trimmable, therefore the overall axial length "L" of distal portion 204 is customizable depending upon the surgical site in which the access device 200 is to be used.

A flange 212 extends radially outwardly from proximal portion 202 of access device 200. The body portion 201 may be shaped via extrusion and mounted to the flange. In other examples, flange 212 may be chemically couplable to proximal portion 202 via use of adhesives or permanently couplable via ultrasonic welding as described above, or may be mounted onto the proximal portion 202 of body portion 201 via mechanical means such as by friction fit, threaded connections, male/female fasteners, snap fit, and other conventional means within the purview of those skilled in the art. Flange 212 is substantially planar and includes a tissue facing surface 208 including an adhesive 210 for securing and sealing the access device within tissue as described above. It is envisioned that flange 212 may be of any suitable shape that is dimensioned to be radially larger than the radial dimension "R" of the body portion 201.

As with the previous example, one or more ports 206 may extend longitudinally through the body portion 201 of access device 200. The port, or ports, 206 are configured to removably receive a surgical object (not show). In examples, access device 200 may include a plurality of ports 206 that are symmetrically arranged with respect to the longitudinal axis "A", as illustrated in FIG. 5. It is further contemplated that each port 206 may be spaced equidistant from the longitudinal axis "A". Ports 206 may be arranged such that they are spaced equally from one another, or alternatively, the distance between adjacent ports 206 may vary.

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope of the disclosure. Additionally, it is envisioned that the elements and features illustrated or described in connection with one exemplary embodiment may be combined with the elements and features of another without departing from the scope of the present disclosure, and that such modifications and variations are also intended to be included within the scope of the present disclosure. Accordingly, the subject matter of the present disclosure is not to be limited by what has been particularly shown and described, except as indicated by the appended claims.

## Claims

1. An access device (100) formed from a compliant material for positioning within a tissue tract for accessing an underlying body cavity, the access device comprising:
a proximal portion (102) comprising a tissue facing surface (108) defining a first radial dimension (D1);
a distal portion (104) extending longitudinally along an axial length, the distal portion defining a second radial dimension (D2) that is substantially uniform along the axial length and smaller than the first radial dimension;
a body portion (101) interconnecting the proximal and distal portions;
at least one port (106) extending through the proximal and distal portions; and
an adhesive (110) for releasably securing and sealing the proximal portion to a tissue surface,
**characterised in that** the body portion (101) comprises a tapering portion (103) adjacent the proximal portion;
the proximal portion (102) exhibits an arcuate configuration and the adhesive (110) is disposed on the tissue facing surface of the arcuate proximal portion (102).

2. The access device of any preceding claim, wherein the adhesive is selected from the group consisting of acrylics, silicones, urethanes, and hydrogels.

3. The access device of any preceding claim, wherein the adhesive is activated or de-activated by an external stimulus.

4. The access device of claim 3, wherein the external stimulus is selected from the group consisting of heat, light, and fluid.

5. The access device of any preceding claim, wherein the adhesive is pressure sensitive.

6. The access device of any preceding claim, wherein the adhesive is coated on the tissue facing surface of the proximal portion.

7. The access device of any of claims 1 to 5, wherein the adhesive is a layer affixed to the tissue facing surface of the proximal portion.

8. The access device of claim 7, wherein the layer further comprises a textured layer including concave surfaces (111) for mechanically securing the tissue facing surface of the proximal portion to the tissue surface.

9. The access device of claim 7, wherein the layer further comprises a textured layer including gecko feet (113) which act as suction pads to mechanically secure the tissue facing surface of the proximal portion to the tissue surface.

10. The access device of any preceding claim, wherein the proximal and distal portions are monolithically formed with the body portion.

11. The access device according to any preceding claim, wherein the at least one port comprises a plurality of ports (206) symmetrically arranged about the longitudinal axis.

12. The access device according to any preceding claim, wherein the compliant material is foam.

## Patentansprüche

1. Zugangseinrichtung (100), die aus einem nachgiebigen Material ist, zur Positionierung innerhalb eines Gewebetraktes für den Zugriff auf eine darunter liegende Körperhöhle, wobei die Zugangseinrichtung umfasst:
einen proximalen Abschnitt (102), der eine dem Gewebe zugewandte Oberfläche (108) aufweist, die eine erste radiale Dimension (D1) definiert:
einen distalen Abschnitt (104), der sich in Längsrichtung entlang einer axialen Länge erstreckt, wobei der distale Abschnitt eine zweite radiale Dimension (D2) definiert, die im Wesentlichen gleichförmig entlang der axialen Länge und kleiner als die erste radiale Dimension ist;
einen Körperabschnitt (101), der den proximalen und den distalen Abschnitt miteinander verbindet;
mindestens eine Öffnung (106), die sich durch die proximalen und distalen Abschnitte erstreckt; und
einen Klebstoff (110) zum lösbaren Befestigen und Abdichten des proximalen Abschnitts an einer Gewebeoberfläche,
**dadurch gekennzeichnet, dass** der Körperabschnitt (101) einen sich verjüngenden Abschnitt (103) angrenzend an den proximalen Abschnitt aufweist;
der proximale Abschnitt (102) eine bogenförmige Konfiguration aufweist und der Klebstoff (110) an der dem Gewebe zugewandten Fläche des bogenförmigen proximalen Abschnitts (102) angeordnet ist.

2. Zugangseinrichtung nach einem der vorhergehenden Ansprüche, wobei der Klebstoff ausgewählt ist aus der Gruppe, die aus Acrylverbindungen, Silikonen, Urethanen und Hydrogelen besteht.

3. Zugangseinrichtung nach einem der vorhergehenden Ansprüche, wobei der Klebstoff durch einen externen Stimulus aktiviert oder deaktiviert wird.

4. Zugangseinrichtung nach Anspruch 3, wobei der externe Stimulus ausgewählt ist aus der Gruppe, die aus Wärme, Licht und Fluid besteht.

5. Zugangseinrichtung nach einem der vorhergehenden Ansprüche, wobei der Klebstoff haftklebend ist.

6. Zugangseinrichtung nach einem der vorgehenden Ansprüche, wobei der Klebstoff auf die dem Gewebe zugewandte Fläche des proximalen Abschnitts aufgetragen ist.

7. Zugangseinrichtung nach einem der Ansprüche 1 bis 5, wobei der Klebstoff eine Schicht ist, die an der dem Gewebe zugewandten Fläche des proximalen Abschnitts befestigt ist.

8. Zugangseinrichtung nach Anspruch 7, wobei die Schicht ferner eine texturierte Schicht umfasst, die gewölbte Oberflächen (111) zur mechanischen Sicherung von der dem Gewebe zugewandten Fläche des proximalen Abschnitts an der Gewebeoberfläche aufweist.

9. Zugangseinrichtung nach Anspruch 7, wobei die Schicht ferner eine texturierte Schicht umfasst, die geckofußartige Elemente (113), die wie Saugnäpfe wirken, zur mechanischen Sicherung von der dem Gewebe zugewandten Fläche des proximalen Abschnitts an der Gewebeoberfläche aufweist.

10. Zugangseinrichtung nach einem der vorhergehenden Ansprüche, wobei die proximalen und distalen Abschnitte einstückig mit dem Körperabschnitt ausgebildet sind.

11. Zugangseinrichtung nach einem der vorhergehenden Ansprüche, wobei die mindestens eine Öffnung eine Mehrzahl von Öffnungen (206) aufweist, die symmetrisch um die Längsachse angeordnet sind.

12. Zugangseinrichtung nach einem der vorhergehenden Ansprüche, wobei das nachgiebige Material Schaumstoff ist.

## Revendications

1. Un dispositif d'accès (100) formé à partir d'un matériau conforme à un positionnement dans un système tissulaire pour accéder à une cavité corporelle sous-jacente, le dispositif d'accès comprenant :
une partie proximale (102) comprenant une surface (108) faisant face au tissus définissant une première dimension radiale (D1) ;
une partie distale (104) s'étendant longitudinalement le long d'une longueur axiale, la partie distale définissant une deuxième dimension radiale (D2) qui est substantiellement uniforme le long de la longueur axiale et est plus petite que la première dimension radiale ;
une partie de corps (101) interconnectant les parties proximales et distales ;
au moins un port (106) traversant les parties proximales et distales ; et
un adhésif (110) pour fixer et sceller de façon amovible la partie proximale à une surface tissulaire,
**caractérisé en ce que** la partie de corps (101) comprend une partie effilée (103) adjacente à la partie proximale ;
la partie proximale (102) présente une configuration arquée et l'adhésif (110) est disposé sur la surface faisant face au tissus de la partie proximale arquée (102).

2. Le dispositif d'accès d'une quelconque revendication précédente, dans lequel l'adhésif est choisi parmi le groupe constitué des acryliques, silicones, uréthanes, et hydrogels.

3. Le dispositif d'accès d'une quelconque revendication précédente, dans lequel l'adhésif est activé ou désactivé par un stimulus externe.

4. Le dispositif d'accès de la revendication 3, dans lequel le stimulus externe est choisi parmi le groupe constitué de chaleur, lumière et fluide.

5. Le dispositif d'accès d'une quelconque revendication précédente, dans lequel l'adhésif est sensible à la pression.

6. Le dispositif d'accès d'une quelconque revendication précédente, dans lequel la surface faisant face au tissu de la partie proximale est recouverte de l'adhésif.

7. Le dispositif d'accès d'une quelconque des revendications 1 à 5, dans lequel l'adhésif est une couche apposée à la surface faisant face au tissu de la partie proximale.

8. Le dispositif d'accès de la revendication 7, dans lequel la couche comprend en outre une couche texturée incluant des surfaces concaves (111) pour fixer mécaniquement à la surface tissulaire la surface faisant face au tissu de la partie proximale.

9. Le dispositif d'accès de la revendication 7, dans lequel la couche comprend en outre une couche texturée incluant des pattes de gecko (113) qui agissent comme des ventouses pour fixer mécaniquement à la surface tissulaire la surface faisant face au tissu de la partie proximale.

10. Le dispositif d'accès d'une quelconque revendication précédente, dans lequel les parties proximales et distales sont formées de façon monolithique avec la partie de corps.

11. Le dispositif d'accès d'une quelconque revendication précédente, dans lequel l'au moins un port comprend une pluralité de ports (206) disposés symétriquement autour de l'axe longitudinal.

12. Le dispositif d'accès d'une quelconque revendication précédente, dans lequel le matériau conforme est une mousse.
